# EUROPÄISCHE PATENTSCHRIFT

(11) **EP 0 696 273 B1**
(45) Veröffentlichungstag und Bekanntmachung des Hinweises auf die Patenterteilung: **03.09.1997**
(21) Anmeldenummer: 94915112.0
(22) Anmeldetag: 25.04.1994
(51) Int. Cl.: C07C 271/60, C08G 18/80

(54) **CARBAMOYLHYDROXYLAMINE AND DEREN VERWENDUNG IN DER POLYMERCHEMIE**
CARBAMOYL HYDROXYLAMINE AND USE THEREOF IN POLYMER CHEMISTRY
CARBAMOYLHYDROXYLAMINE ET SON UTILISATION DANS LA CHIMIE DES POLYMERES

(30) Priorität: 04.05.1993 DE 4314623
(43) Veröffentlichungstag der Anmeldung: 14.02.1996
(73) Patentinhaber: BASF AKTIENGESELLSCHAFT, 67056 Ludwigshafen (DE)
(72) Erfinder: BAUER, Gerhard, D-69469 Weinheim (DE); HAEBERLE, Karl, D-67346 Speyer (DE); BAUMSTARK, Roland, D-67434 Neustadt (DE); BOTT, Kaspar, D-68165 Mannheim (DE)
(86) Internationale Anmeldenummer: EP9401283
(87) Internationale Veröffentlichungsnummer: WO9425433

(56) Entgegenhaltungen:
- EP-A- 0 244 355
- DE-A- 3 807 555

## Beschreibung

Die Erfindung betrifft eine Verbindung der Formel 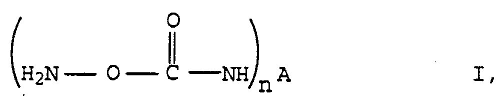 welche auch als Salz vorliegen kann, worin A für einen n-wertigen organischen Rest steht und n eine ganze Zahl größer 1 ist.

Des weiteren betrifft die Erfindung Dispersionen oder Lösungen von radikalischen Polymerisaten, Polykondensaten oder Polyaddukten, welche Verbindungen der Formel I enthalten.

Bei Copolymerisaten, welche in Beschichtungsmitteln oder Klebstoffen Verwendung finden, handelt es sich vielfach um vernetzungsfähige Copolymerisate. Durch eine Vernetzung können z.B. Schutzüberzüge oder Klebstoffbeschichtungen mit guten elastischen Eigenschaften, hoher Kohäsion, d.h. innere Festigkeit, hoher Chemikalien- und Lösemittelbeständigkeit erhalten werden.

Zur Vernetzung wird den Copolymerisaten im allgemeinen ein Vernetzungsmittel zugesetzt, das mit funktionellen Gruppen im Copolymerisat reagiert.

Mögliche Vernetzungsmittel sind z.B. Polyisocyanate, welche mit Hydroxyl- oder Aminogruppen reagieren.

Aus der DE-A-35 21 618 sind entsprechende waßrige Klebstoffzubereitungen bekannt, bei denen in Wasser dispergierte Polyisocyanate wäßrigen Dispersionen radikalische polymerisierter Copolymerisate als Vernetzungsmittel zugesetzt werden. Ähnliche Klebstoffzubereitungen sind auch in der US-A 4 396 738 und DE-A-31 12 117 beschrieben.

Nachteilig bei diesen wäßrigen Zubereitungen ist jedoch die mangelnde Lagerstabilität. Das Polyisocyanat darf daher erst kurz vor seiner Verwendung als Vernetzungshilfsmittel in Wasser dispergiert und mit dem Copolymerisat gemischt werden.

Eine erhöhte Lagerstabilität kann durch Umsetzung der Isocyanatgruppen mit Blockierungsmitteln, z.B. Oximen, Caprolactam, Phenolen, Maleinsäuredialkylestern erreicht werden. Die erhaltenen sog. blockierten Polyisocyanate hydrolysieren in wäßriger Dispersion nur noch in untergeordnetem Ausmaß.

Gegenstand der DE-A-38 07 555 ist ein solches, mit Oximen blokkiertes Diisocyanat, welches in Wasser dispergiert wird und sich als Zusatz für in Wasser dispergierte Polymerisate eignet.

Vernetzungsreaktionen treten jedoch erst nach Abspaltung des Blockierungsmittels bei Temperaturen ab ca. 130°C auf.

Bisher bekannte wäßrige Klebstoff zubereitungen mit Polyisocyanaten als Vernetzungshilfsmittel sind daher entweder nicht lagerstabil und können daher nur als 2-Komponentensystem Verwendung finden oder vernetzen erst bei hohen Temperaturen.

Lagerstabile, bei Raumtemperatur nach Entfernen des Lösungsmittels vernetzende wäßrige Dispersionen sind aus der EP-A-3516 bekannt. Diese Dispersionen enthalten Polyhydrazide, welche mit im Copolymerisat einpolymerisierten Monomeren mit Carbonylgruppen reagieren.

Aus der deutschen Patentanmeldung P 42 19 384.2 sind Oximether als Vernetzungsmittel bekannt. In der EP-A-516 074 sind Aminooxyderivate als Vernetzer für Keto- oder Aldehydgruppen enthaltende Copolymerisate beschrieben.

In der EP-A-522 306 sind mit Oximen blockierte Polyisocyanate als Vernetzer für Carbonylgruppen enthaltende Polymerisate beschrieben.

Grundsätzlich besteht ein Bedarf an weiteren, bei Raumtemperatur vernetzenden Dispersionen, um Alternativen zur Polyhydrazidvernetzung zu Verfügung stellen zu können. Des weiteren sollen diese Dispersionen gute anwendungstechnische Eigenschaften, z.B. eine gute Haftung, insbesondere Naßhaftung auf unterschiedlichsten Substraten, aufweisen.

Aufgabe der vorliegenden Erfindung waren daher lagerstabile Dispersionen oder Lösungen von vernetzbaren Copolymerisaten, welche ein Vernetzungsmittel enthalten und bei Raumtemperatur vernetzbar sind.

Demgemäß wurde die oben definierte Verbindung, sowie Dispersionen oder Lösungen, welche diese Verbindung enthalten, gefunden.

Die Verbindung der Formel I eignet sich als Vernetzungsmittel oder Haftungsvermittler in Dispersionen oder Lösungen von radikalischen Polymerisaten, Polykondensaten oder Polyaddukten.

Die Vernetzung von Keto- oder Aldehydgruppen enthaltenden radikalischen Polymerisaten, Polykondensaten oder Polyaddukten mit Vernetzungsmitteln der Formel I tritt bei Entfernung der flüssigen Phase der Dispersion oder Lösung ein.

In Formel I steht A für einen n-wertigen organischen Rest. Vorzugsweise steht A für einen aliphatischen oder araliphatischen Kohlenwasserstoffrest mit 4 bis 20, vorzugsweise 4 bis 10 Kohlenstoffatomen. Ganz besonders bevorzugt steht A für einen aliphatischen Rest.

A kann auch für einen organischen Rest stehen, welcher z.B. Urethangruppen, Harnstoffgruppen, Biuretgruppen, Isocyanuratgruppen oder Uretdiongruppen enthält und vorzugsweise ein Molekulargewicht bis zu 10000 g/Mol, besonders bevorzugt bis zu 1000 g/ Mol hat. A leitet sich generell ab von Diisocyanaten oder Polyisocyanaten, d.h. A entspricht den Di- bzw. Polyisocyanaten unter Weglassen der Isocyanatgruppen.

n steht vorzugsweise für eine ganze Zahl von 2 bis 20, besonders bevorzugt von 2 bis 10 und ganz besonders bevorzugt von 2 bis 4.

Ausgangskomponente für die Herstellung der Verbindung I sind Polyisocyanate.

Als Polyisocyanate genannt seien z.B. Diisocyanate wie Tetramethylendiisocyanat, Hexamethylendiisocyanat (HDI), Dodecamethylendiisocyanat, 1,4-Diisocyanatocyclohexan, 4,4'-Di-(isocyanatocyclohexyl)-methan (HMDI), Trimethylhexandiisocyanat, 1-Isocyanato-3,3,5-trimethyl-5-isocyanatocyclohexan (IPDI), 2,4- und 2,6-Diisocyanatotoluol (TDI), Tetramethylxylylendiisocyanat, p-Xylylendiisocyanat, 2,4'- und 4,4'-Diisocyanatodiphenylmethan sowie z.B. Isocyanurat- oder Biuretgruppen aufweisende Polyisocyanate, insbesondere solche auf Basis von 1,6-Diisocyanatohexan und/oder von l-Isocyanato-3,3,5-trimethyl-5-isocyanatomethylcyclohexan oder Umsetzungsprodukte der Polyisocyanate mit mehrwertigen, insbesondere 2- bis 5-wertigen Alkoholen. Bevorzugt sind aliphatische Alkohole mit insgesamt 2 bis 8 C-Atomen, wie Ethylenglykol, Butandiol-1,4, Propandiol-1,2, Glycerin, Trimethylolpropan oder Pentaerythrit.

Ebenfalls geeignet sind hydrophil modifizierte Polyisocyanate. Diese sind selbstdispergierbar in Wasser, so daß zur Dispergierung auf Emulgatoren und Dispergierhilfsmittel weitgehend verzichtet werden kann. Bekannt sind selbstdispergierbare Polyisocyanate mit nicht ionischen hydrophilen Gruppen, insbesondere Umsetzungsprodukte von Polyisocyanaten mit Polyalkylenetheralkoholen, wie sie z.B. in der EP-A-206 059 beschrieben sind. Auch durch Einbau von ionischen Gruppen bzw. von in ionische Gruppen überführbaren Gruppen werden Polyisocyanate selbstdispergierbar. Letztere sind z.B. aus der DE-A-41 13 160 und DE-A-40 01 783 bekannt.

Bevorzugt eingesetzt werden Hexamethylendiisocyanat, IPDI und HMDI und Gemische daraus, sowie die davon abgeleiteten höherfunktionellen Derivate.

Die Isocyanatgruppen der Polyisocyanate werden mit Oximen bzw. Hydroxamsäureestern gemäß den bekannten Blockierungsreaktionen 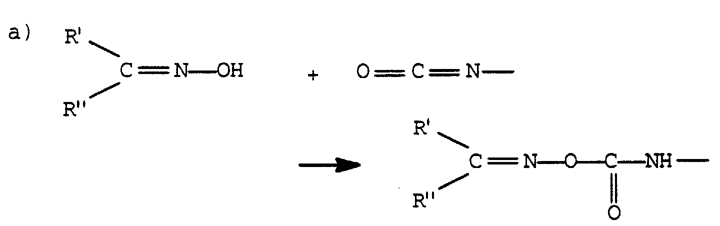 bzw. 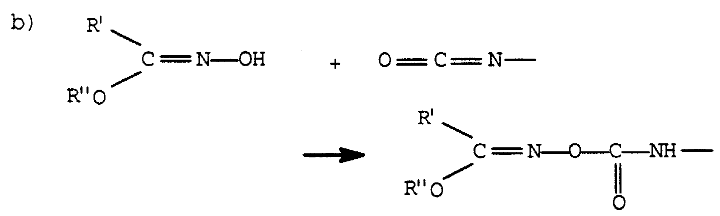 blockiert.

Als Oxime sind solche von aliphatischen, cycloaliphatischen oder aromatischen Aldehyden oder Ketonen, z.B. Acetonoxim, Methylethylketonoxim, Diethylketoxim, Methylisopropylketoxim, Methylisobutylketoxim, Diisopropylketoxim, Cyclohexanonoxim, 2-Methylcyclohexanonoxim, 2,6-Dimethylcyclohexanonoxim, Acetophenonoxim, Benzophenonoxim oder Diethylglyoxim geeignet. Bevorzugt sind Oxime von aliphatischen Ketonen mit einer Ketogruppe und insgesamt 3 bis 12 Kohlenstoffatomen, insbesondere Acetonoxim und Methylethylketonoxim. Als Hydroxamsäureester sind solche bevorzugt, bei denen die obigen Variablen R' und R" jeweils für eine C₁-C₅-Alkylgruppe stehen, wobei R' auch für ein H-Atom stehen kann.

Die Herstellung der blockierten Polyisocyanate kann in dem Fachmann bekannter Weise in inerten organischen Lösungsmitteln, z.B. aromatischen Kohlenwasserstoffen wie Toluol, oder ohne Lösungsmittel bei Temperaturen zwischen 20 und 150°C, bevorzugt 20 und 100°C erfolgen.

Bevorzugt beträgt das Verhältnis von Isocyanatgruppen der eingesetzten Polyisocyanate zu den Oximen bzw. Hydroxamsäureestern 1:0,9 bis 1:1,4. Besonders bevorzugt liegt es zwischen 1:0,95 und 1:1,1.

Die Blockierungsreaktion kann zweckmäßigerweise in Gegenwart von Metallsalzen als Katalysatoren, z.B. Dibutylzinndilaurat oder Zinnoctoat durchgeführt werden.

Anschließend werden die blockierten Polyisocyanate hydrolytisch in Gegenwart von Wasser und starken Säuren mit einer Säurekonstanten > 10⁻², bevorzugt > 10⁻¹ (s. Anorganikum, Berlin 1977, Seite 458) in die Verbindungen der Formel I gespalten. Als Säure wird besonders bevorzugt Chlorwasserstoff verwendet. Die als Spaltprodukte erhaltenen Verbindungen der Formel I fallen hierbei als Salze bzw. Hydrochloride an (Aminogruppen sind protoniert). Die freien Verbindungen der Formel I können, soweit gewünscht durch geeignete Maßnahmen zur Deprotonierung, z.B. durch Zugabe von Basen, erhalten werden. Die Spaltreaktion wird vorzugsweise bei Reaktionstemperaturen von 0 bis 60°C, bevorzugt 20 bei 35°C, durchgeführt. Die Reaktionstemperatur kann gegenüber sonst üblichen Temperaturen bei hydrolytischen Spaltungen von z.B. Oximethern, überraschend niedrig gehalten werden (s.a. Houben Weyl, Methoden der organischen Chemie, Bd. 10, 1 Seite 1186).

Verwendet man zur Herstellung der Verbindungen mit der Struktur I die durch Oxime blockierten Polyisocyanate, so ist es vorteilhaft, das bei der Spaltung gebildete Keton oder Aldehyd aus dem Reaktionsgemisch durch Verdampfung zu entfernen, um eine zufriedenstellenden Umsatz zu erzielen. Bei Verwendung von mit Hydroxamsäureester blockierten Polyisocyanaten liegt das Reaktionsgleichgewicht im allgemeinen ganz auf der Seite der Spaltprodukte, so daß eine Abtrennung der Ketone bzw. Aldehyde kaum zusätzlich Vorteile bringt. Vorteilhaft ist die Zugabe eines Lösungsmittels bzw. Fällungsmittels, z.B. Diethylether, in dem das Produkt schwer löslich ist und so ausgefällt werden kann.

Die Verbindung der Formel I kann Dispersionen oder Lösungen von radikalischen Polymerisaten, Polyadduktion oder Polykondensaten als Vernetzungsmittel oder zur Verbesserung der Haftung zugegeben werden.

Die Verbindung der Formel I eignet sich insbesondere als Vernetzungsmittel für ein Polymerisat, Polykondensat oder Polyaddukt, das zu 0,001 bis 20 Gew.-%, bevorzugt 0,01 bis 10 Gew.-% und ganz besonders bevorzugt zu 0,05 bis 4 Gew.-% aus Aldehydgruppen -CHO- oder Ketogruppen -CO- besteht und in Lösung oder Dispersion vorliegt.

Es kann sich dabei z.B. um ein radikalisch polymerisiertes Copolymerisat, ein Polyester als Polykondensat oder ein Polyurethan als Polyaddukt handeln.

Im Falle der radikalisch polymerisierten Copolymerisate sind die Aldehyd- oder Ketogruppen vorzugsweise durch ethylenisch ungesättigte Verbindungen, welche diese Gruppen enthalten, einpolymerisiert.

Vorzugsweise handelt es sich um ethylenisch ungesättigte Verbindungen mit ein oder zwei Aldehyd-, bzw. Ketogruppen oder einer Aldehyd- und einer Ketogruppe und einer radikalisch polymerisierbaren olefinischen Doppelbindung (im weiteren Monomere a) genannt).

Im Falle eines Polyesters kann es sich z.B. um Monoalkohole, Diole, Monocarbonsäuren oder Dicarbonsäuren, im Falle eines Polyurethans kann es sich z.B. um Mono-, Diisocyanate oder ebenfalls Monoalkohole oder Diole handeln, welche Aldehyd- oder Ketogruppen enthalten.

Als Monoalkohole genannt seien z.B. Hydroxyaceton, Hydroxybenzaldehyd, Acetoin und Benzoin.

Geeignete Monocarbonsäuren sind z.B. Ketocarbonsäuren, wie Brenztraubensäure oder Lävulinsäure.

Bekannt sind ketogruppenhaltige Polyurethandispersionen z.B. aus DE-A-38 37 519, EP-A-332 326 und EP-A-442 652.

Ferner können Verbindungen mit Aldehyd- oder Ketogruppen in die Polymerisate, Polykondensate oder Polyaddukte nicht nur als Bestandteil der Hauptkette, sondern auch durch Umsetzung mit reaktiven Gruppen in der Polymerhauptkette an die Polymerisate, Polykondensate oder Polyaddukte gebunden werden.

Bevorzugt ist ein radikalisch polymerisiertes Copolymerisat, welches aus den Monomeren a), welche Aldehyd- oder Ketogruppen enthalten, und weiteren Monomeren b) und c) besteht.

Als Monomere a) kommen z.B. Acrolein, Methacrolein, Vinylalkylketone mit 1 bis 20, vorzugsweise 1 bis 10 Kohlenstoffatomen im Alkylrest, Formylstyrol, (Meth-)acrylsäurealkylester mit ein oder zwei Keto- oder Aldehyd-, bzw. einer Aldehyd- und einer Ketogruppe im Alkylrest, wobei der Alkylrest vorzugsweise insgesamt 3 bis 10 Kohlenstoffatome umfaßt, z.B. (Meth)acryloxyalkylpropanale, wie sie in der DE-A-27 22 097 beschrieben sind. Des weiteren eignen sich auch N-Oxoalkyl(meth)acrylamide wie sie z.B. aus der US-A-4 226 007, der DE-A-20 61 213 oder DE-A-22 07 209 bekannt sind.

Besonders bevorzugt sind Acetoacetyl(meth)acrylat, Acetoacetoxyethyl(meth)acrylat und insbesondere Diacetonacrylamid.

Die Hauptmonomeren b) sind in dem Copolymerisat insbesondere zu 20 bis 99,99, bevorzugt 60 bis 99,9 und besonders bevorzugt zu 80 bis 99,5 Gew.-%, bezogen auf das Copolymerisat, enthalten.

Als Monomere b) kommen Ester der Acryl- oder Methacrylsäure von 1 bis 20 C-Atome enthaltenden Alkylalkoholen in Betracht. Als solche Alkohole seien genannt Methanol, Ethanol, n- oder i-Propanol, n-, s- und t-Butanol, n-Pentanol, Isoamylalkohol, n-Hexanol, Octanol, 2-Ethylhexanol, Lauryl- und Stearylalkohol.

Gute Ergebnisse werden mit (Meth)-acrylsäurealkylestern mit einem C₁-C₁₀-Alkylrest, wie Methylmethacrylat, Methylacrylat, n-Butylacrylat, Ethylacrylat und 2-Ethylhexylacrylat erzielt.

Insbesondere sind auch Mischungen der (Meth)acrylsäurealkylester geeignet.

Weiterhin kommen Vinylester von Carbonsäuren mit 1 bis 20 C-Atomen wie Vinyllaurat, -stearat, Vinylpropionat und Vinylacetat in Betracht.

Als vinylaromatische Verbindungen mit bis zu 20 C-Atomen kommen Vinyltoluol, α- und p-Styrol, α-Butylstyrol, 4-n-Butylstyrol, 4-n-Decylstyrol und vorzugsweise Styrol in Betracht. Beispiele für ethylenisch ungesättigte Nitrile sind Acrylnitril und Methacrylnitril.

Die Vinylhalogenide sind mit Chlor, Fluor oder Brom substituierte ethylenisch ungesättigte Verbindungen, bevorzugt Vinylchlorid und Vinylidenchlorid.

Als nicht aromatische Kohlenwasserstoffe mit 2 bis 8 C-Atomen und mindestens zwei konjugierten olefinischen Doppelbindungen seien Butadien, Isopren und Chloropren genannt.

Die Monomeren b) können insbesondere auch im Gemisch eingesetzt werden, vor allem, um gewünschte Glasübergangstemperaturen des Copolymerisats einzustellen.

Als weitere, d.h. nicht unter a) und b) fallende, copolymerisierbare Monomere c) kommen z.B. Ester der Acryl- und Methacrylsäure von Alkoholen mit 1 bis 20 C-Atomen, die außer dem Sauerstoffatom in der Alkoholgruppe mindestens ein weiteres Heteroatom enthalten und/oder die einen aliphatischen oder aromatischen Ring enthalten, in Betracht.

Genannt seien z.B. 2-Ethoxyethylacrylat, 2-Butoxyethyl(meth)acrylat, Dimethylaminoethyl(meth)acrylat, Diethylaminoethyl(meth)acrylat, (Meth)acrylsäurearyl-, -alkaryl- oder cycloalkylester, wie Cyclohexyl(meth)acrylat, Phenylethyl(meth)-acrylat, Phenylpropyl(meth)acrylat- oder Acrylsäureester von heterocyclischen Alkoholen wie Furfuryl- (meth)acrylat.

Darüber hinaus seien noch weitere Comonomere wie (Meth)acrylamid sowie deren am Stickstoff mit C₁-C₄-Alkyl substituierten Derivate genannt.

Von besonderer Bedeutung sind auch hydroxyfunktionelle Comonomere, z.B. (Meth)acrylsäure-C₁-C₁₅-alkylester, welche durch ein oder zwei Hydroxygruppen substituiert sind. Insbesondere von Bedeutung als hydroxyfunktionelle Comonomere sind (Meth)acrylsäure-C₁-C₈-Hydroxyalkylester, wie n-Hydroxyethyl-, n-Hydroxypropyl- oder n-Hydroxybutyl(meth)acrylat.

Die Mitverwendung von Comonomeren mit salzbildenden Gruppen empfiehlt sich z.B. für die Herstellung selbstdispergierbarer Copolymerisate, die z.B. für wäßrige Sekundärdispersionen geeignet sind. Comonomere mit salzbildenden Gruppen sind insbesondere Itaconsäure, Acrylsäure und Methacrylsäure.

Der Gewichtsanteil der weiteren Comonomeren im Copolymerisat kann insbesondere 0 bis 50 %, vorzugsweise 0 bis 20 % und ganz besonders bevorzugt 0 bis 10 Gew.% betragen.

Die Anteile der Monomeren a), b) und c) addieren sich zu 100 Gew.-%.

Der Anteil der Monomeren a) wird dabei so gewählt, daß der oben angegebene Gehalt an Aldehyd- oder Ketogruppen im Copolymerisat vorliegt.

Die Herstellung des Copolymerisats erfolgt im allgemeinen durch radikalische Polymerisation. Geeignete Polymerisationsmethoden, wie Substanz-, Lösungs-, Suspensions- oder Emulsionspolymerisation sind dem Fachmann bekannt.

Vorzugsweise wird das Copolymerisat durch Lösungspolymerisation mit anschließender Dispergierung in Wasser oder besonders bevorzugt durch Emulsionspolymerisation hergestellt, wobei das Copolymerisat als wäßrige Dispersion erhalten wird.

Die Comonomeren können bei der Emulsionspolymerisation wie üblich in Gegenwart eines wasserlöslichen Initiators und eines Emulgators bei vorzugsweise 30 bis 95°C polymerisiert werden.

Geeignete Initiatoren sind z.B. Natrium-, Kalium- und Ammoniumpersulfat, tert.-Butylhydroperoxide, wasserlösliche Azoverbindungen oder auch Redoxinitiatoren.

Als Emulgatoren dienen z.B. Alkalisalze von längerkettigen Fettsäuren, Alkylsulfate, Alkylsulfonate, alkylierte Arylsulfonate oder alkylierte Biphenylethersulfonate.

Des weiteren kommen als Emulgatoren Umsetzungsprodukte von Alkylenoxiden, insbesondere Ethylen- oder Propylenoxid mit Fettalkoholen, -säuren oder Phenol, bzw. Alkylphenolen in Betracht.

Im Falle von wäßrigen Sekundärdispersionen wird das Copolymerisat zunächst durch Lösungspolymerisation in einem organischen Lösungsmittel hergestellt und anschließend unter Zugabe von Salzbildnern, z.B. von Ammoniak zu Carbonsäuregruppen enthaltenden Copolymerisaten, in Wasser ohne Verwendung eines Emulgators oder Dispergierhilfsmittels dispergiert. Das organische Lösungsmittel kann abdestilliert werden. Die Herstellung von wäßrigen Sekundärdispersionen ist dem Fachmann bekannt und z.B. in der DE-A-37 20 860 beschrieben.

Zur Einstellung des Molekulargewichts können bei der Polymerisation Regler eingesetzt werden. Geeignet sind z.B. -SH enthaltende Verbindungen wie Mercaptoethanol, Mercaptopropanol, Thiophenol, Thioglycerin, Thioglykolsäureethylester, Thioglykolsäuremethylester und tert.-Dodecylmercaptan.

Die Art und Menge der Comonomeren wird zweckdienlicherweise so gewählt, daß das erhaltene Copolymerisat eine Glasübergangstemperatur zwischen bevorzugt -60 und +140°C. Je nachdem, ob harte oder weiche Beschichtungen gewünscht sind, werden durch Wahl der Monomeren hohe oder tiefe Glasübergangstemperaturen eingestellt. Die Glasübergangstemperatur des Copolymerisats läßt sich nach üblichen Methoden wie Differentialthermoanalyse oder Differential Scanning Calorimetrie (s. z.B. ASTM 3418/82, sog. "midpoint temperature") bestimmen.

Die Dispersion oder Lösung enthält als Haftungsvermittler oder als Vernetzungsmittel eine Verbindung der Formel I.

Vorzugsweise beträgt der Gewichtsanteil der Verbindung der Formel I in den Dispersionen der Lösungen 0,01 bis 30 Gew.-%, besonders bevorzugt 0,1 bis 20 Gew.-%, ganz besonders bevorzugt 0,1 bis 5 Gew.-%, bezogen auf das Polymerisat, Polykondensat oder Polyaddukt. Vorteilhafterweise wird der Gehalt im Falle Keto- oder Aldehydgruppen enthaltender radikalischer Polymerisate, Polykondensate oder Polyaddukte so gewählt, daß die Gruppen 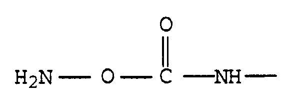 im ungefähr äquimolaren Verhältnis zu den Keto- und/oder Aldehydgruppen vorliegen.

Metallsalze oder Metallkomplexe, welche in den Dispersionen oder Lösungen vorhanden sind bzw. zugesetzt werden, beeinträchtigen die Vernetzung oder Haftungsverbesserung durch Verbindungen I im allgemeinen nicht.

Der Feststoffgehalt der erfindungsgemäßen Dispersion oder Lösung liegt bevorzugt zwischen 20 und 90 Gew.-%, insbesondere zwischen 30 und 70 Gew.-%.

Die erfindungsgemäßen Dispersionen oder Lösungen eignen sich als Beschichtungsmittel für unterschiedliche Substrate mit Kunststoff-, Holz- oder Metalloberflächen oder z.B. für Textilien, Fliesstoffe, Leder oder Papier. Sie eignen sich ebenfalls für Anwendungen in der Bauchemie z.B. als Klebstoffe, Dichtungsmassen, Bindemittel oder ähnliches. Bei den Beschichtungen kann es sich z.B. um Anstriche, Schutzüberzüge oder Klebstoffbeschichtungen handeln.

Insbesondere ist für die genannten Verwendungen eine wäßrige Dispersion eines radikalisch polymerisierten, insbesondere eines Keto- und/oder Aldehydgruppen enthaltenden Copolymerisats geeignet.

Die wäßrige Dispersion kann auch organische, vorzugsweise mit Wasser mischbare Lösungsmittel als Hilfslösungsmittel enthalten.

Die erfindungsgemäße Dispersion oder Lösung kann je nach Verwendungszweck übliche Hilfs- und Zusatzmittel enthalten. Hierzu gehören beispielsweise Füllstoffe wie Quarzmehl, Quarzsand, hochdisperse Kieselsäure, Schwerspat, Calciumcarbonat, Kreide, Dolomit oder Talkum, die oft zusammen mit geeigneten Netzmitteln wie z.B. Polyphosphaten wie Natriumhexamethaphosphat, Naphthalinsulfonsäure, Ammonium- oder Natriumpolyacrylsäuresalze eingesetzt werden, wobei die Netzmittel im allgemeinen von 0,2 bis 0,6 Gew.-%, bezogen auf den Füllstoff, zugesetzt werden.

Fungizide zur Konservierung werden, falls gewünscht im allgemeinen in Mengen von 0,02 bis 1 Gew.-%, bezogen auf die gesamte Dispersionen oder Lösung eingesetzt. Geeignete Fungizide sind beispielsweise Phenol- oder Kresol-Derivate oder zinnorganische Verbindungen.

Besonders eignet sich die erfindungsgemäßen Dispersionen oder Lösungen, insbesondere als wäßrige Dispersion eines radikalischen Copolymerisats, als Dichtstoff oder Klebstoff, insbesondere z.B. als Kaschierklebstoff zur Herstellung von Verbundfolien und Glanzfolien. Als Klebstoff können die Dispersionen neben obengenannten Zusatzstoffen noch spezielle, in der Klebstofftechnologie übliche Hilfs- und Zusatzmittel enthalten. Hierzu gehören beispielsweise Verdickungsmittel, Weichmacher oder auch klebrigmachende Harze wie z.B. Naturharze oder modifizierte Harze wie Kolophoniumester oder synthetische Harze wie Phthalatharze.

Dispersionen, welche als Klebstoff Verwendung finden, enthalten besonders bevorzugt Alkyl(meth)acrylate als Comonomere b) im Copolymerisat.

Die Glasübergangstemperatur der Copolymerisate wird bei der Verwendung als Klebstoffzubereitung bevorzugt auf Werte zwischen 0 und -40°C eingestellt.

Die Dispersionen zeigen bei der Verwendung als Klebstoff bzw. als Bindemittel in Anstrichen überraschend auch eine sehr gute Haftung, insbesondere Naßhaftung.

Der pH-Wert der Dispersion wird bevorzugt auf einen Wert zwischen 2 und 9 eingestellt, da die Vernetzungsreaktion mit den Copolymerisaten sauer katalysiert werden kann.

Die erfindungsgemäße Dispersionen oder Lösungen, welche Verbindungen der Formel I enthalten, sind lagerstabil. Die Vernetzungsreaktion, z.B. mit Keto- und/oder Aldehydgruppen, tritt schon bei Raumtemperatur bei Entfernen der flüssigen Phase, z.B. Verflüchtigung des Wassers ein.

Durch Temperaturerhöhung z.B. auf 30 bis 100°C kann die Verflüchtigung des Wassers beschleunigt werden.

Bei der Beschichtung von Substraten ist es prinzipiell auch möglich, eine Dispersion oder Lösung des Polymerisats, Polykondensats oder Polyaddukts, welche die Verbindung der Formel I nicht enthält, auf eine Oberfläche aufzutragen, auf die bereits vorher in einem getrennten Arbeitsgang Verbindungen der Formel I aufgebracht wurden.

In diesem Fall wirken die Verbindungen als sog. Primer.

Nach Auftragen der Dispersion oder Lösung tritt dann die Vernetzung ein oder zeigt sich eine Haftungsverbesserung.

### Herstellung der Copolymerisatdispersionen

### Dispersion 1

In einem Reaktionsgefäß mit Rührer und zwei Zulaufgefäßen (Zulauf 1 und Zulauf 2) wurden 400 g entsalztes Wasser, 150 g des Zulaufs 1 (siehe unten), 25 g einer 20 gew.-%igen wäßrigen Lösung eines mit 18 Ethylenoxideinheiten veretherten Fettalkohols (C₁₆/C₁₈), 5 g einer 20 gew.-%igen wäßrigen Lösung des Dinatriumsalzes von p-Dodecyldiphenyletherdisulfonat und 50 g des Zulaufs 2 vorgelegt und auf 85°C erwärmt. Nach 15 Minuten wurde innerhalb von 2 h gleichmäßig der Zulauf 1 sowie innerhalb von 2,25 h gleichmäßig der Zulauf 2 dem Reaktionsgefäß zugegeben.

Nach der vollständigen Initiatorzugabe (Zulauf 2) wurde die Dispersion noch 2 h bei 85°C gerührt.

| Zulauf 1: (wird während der Polymerisation gerührt) | |
|---|---|
| 368 g | entsalztes Wasser |
| 50 g | 20 gew.-%ige wäßrige Lösung eines mit 18 Ethylenoxideinheiten veretherten Fettalkohols (C₁₆/C₁₈) (Emulgator) |
| 75 g | 20 gew.-%ige wäßrige Lösung des Dinatriumsalzes von Dodecyldiphenyletherdisulfonat (Emulgator) |
| 38 g | 2-(Acetoacetoxy)ethylmethacrylat (AAEM) |
| 30 g | 50 gew.-%ige wäßrige Lösung von Acrylamid |
| 27 g | Methacrylsäure |
| 300 g | Methylmethacrylat |
| 700 g | n-Butylacrylat |

| Zulauf 2: | |
|---|---|
| 200 g | entsalztes Wasser |
| 5 g | Natriumpersulfat |

### Dispersion 2

Die Herstellung der Dispersion 2 entsprach Dispersion 1 mit der Ausnahme, daß das Methylmethacrylat in Zulauf 1 vollständig gegen Styrol ausgetauscht wurde.

Herstellung des Vernetzers (V1) 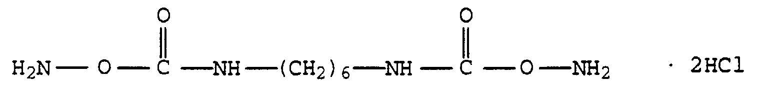
a) Blockierung von Hexamethylendiisocyanat mit Acetonoxim
   146,2 g (2,0 mol) Acetonoxim wurden in 600 g Aceton gelöst. Dann wurden binnen 30 min 168 g (1,0 mol) Hexamethylendiisocyanat zugetropft. Nach Rühren über Nacht wurden 190,3 g Produkt abgesaugt (Schmelzpunkt 94-96°C).
b) Hydrolytische Spaltung zu O,O'-[(N,N'-Hexamethylen)-bis-carbamoyl]-hydroxylammoniumchlorid
   Eine Mischung aus 78,5 g (0,25 mol) O,O'-[(N,N'-Hexamethylen)-bis-carbamoyl]-acetonoxim (Produkt aus a)), 150 g konz. Salzsäure und 50 g Wasser wurde 5 h im Wasserstrahlvakuum (20-30 mbar) auf 35-38°C erhitzt. Anschließend destillierte man die überschüssige Salzsäure und das Wasser mit 300 ml 1,2-Dichlorethan als Schleppmittel im Vakuum (210 mbar) bei 37°C innerhalb 3 h ab. Das im Schleppmittel suspendierte Produkt wurde abfiltriert und an der Luft getrocknet; Ausbeute 69,8 G (91 % d. Theorie), Schmp. 114°C (Zers.).

| Elementaranalyse: | | | | | |
|---|---|---|---|---|---|
| | C | H | O | N | Cl |
| Ber. | 31,28 | 6,56 | 20,83 | 18,24 | 23,08 |
| Gef. | 30,9 | 6,9 | 21,1 | 17,7 | 23,1 |

Die Struktur der Verbindung wurde außerdem anhand des ¹H-NMR-Spektrums sichergestellt.

Prüfung der Vernetzungsfähigkeit:

Zu je 100 g der Dispersionen 1 und 2 wurden folgende Mengen einer 20 gew.-%igen wäßrigen Lösung des Vernetzers (V1) zugegeben (jeweils a-c in der Tabelle) und der End-pH mit konzentriertem wäßrigen Ammoniak eingestellt.

| Dispersion | Vernetzermenge (20 gew.-%ige Lösung) [g] |
|---|---|
| 1 | - |
| 1a | 9,0 |
| 1b | 6,8 |
| 1c | 3,4 |
| 2 | - |
| 2a | 9,0 |
| 2b | 6,8 |
| 2c | 3,4 |

Die Dispersionen wurden 4 Tage bei 23°C und 50 % Luftfeuchtigkeit verfilmt. Aus den 500 µm dicken Filmen wurden ca. 4 cm² große Stücke geschnitten und diese über einen Zeitraum von 24 h bei Raumtemperatur in 100 ml Tetrahydrofuran (THF) gequollen.

Die Lösungsmittelaufnahme der Probenkörper wurde gravimetrisch ermittelt und wird in Prozent angegeben. Je niedriger dabei die Quellwerte sind, desto stärker ist die Vernetzung der Proben.

### Beispiel 1

| Nr. | Molares Verhältnis Vernetzer/AAEM | pH | THF-Quellung nach 24 h in [Gew.-%] |
|---|---|---|---|
| 1 | kein Vernetzer | 6 | 1350 |
| 1a | 0,74:1 | 4 | 580 |
| | | 6 | 530 |
| | | 8 | 560 |
| 1b | 0,55:1 | 4 | 650 |
| | | 6 | 665 |
| | | 8 | 605 |
| 1c | 0,28:1 | 4 | 950 |
| | | 6 | 1020 |
| | | 8 | 815 |
| 2 | kein Vernetzer | 6 | 1060 |
| 2a | 0,74:1 | 6 | 495 |
| 2b | 0,55:1 | 6 | 545 |
| 2c | 0,28:1 | 6 | 880 |

## Patentansprüche

1. Verbindung der Formel 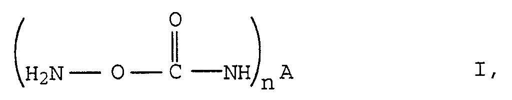 welche auch als Salz vorliegen kann, worin A für einen n-wertigen organischen Rest steht und n eine ganze Zahl größer 1 ist.

2. Verfahren zur Herstellung einer Verbindung der Formel I, dadurch gekennzeichnet, daß Polyisocyanate, deren Isocyanatgruppen durch Oxime, bzw. Hydroxamsäureester, blockiert sind, mit Wasser und einer Säure mit einer Säurekonstante > 10⁻² umgesetzt werden.

3. Dispersion oder Lösung eines radikalischen Polymerisats, Polykondensats oder Polyaddukts, enthaltend eine Verbindung der Formel I.

4. Dispersion oder Lösung eines radikalischen Polymerisats, Polykondensats oder Polyaddukts, welches zu 0,001 bis 20 Gew.-% aus Aldehydgruppen - CHO - oder Ketogruppen - CO - besteht, enthaltend eine Verbindung der Formel I.

5. Dispersion oder Lösung nach Anspruch 3 oder 4, enthaltend als radikalisches Polymerisat Copolymerisate, wobei das Copolymerisat aus
a) mindestens einem Comonomeren mit mindestens einer Aldehyd- oder Ketogruppe,
b) 20 bis 99,99 Gew.-% mindestens eines C₁- bis C₂₀-Alkyl(meth)acrylats, eines Vinylesters von 1 bis 20 C-Atome enthaltenden Carbonsäuren, eines Vinylaromaten mit bis zu 20 C-Atomen, eines ethylenisch ungesättigten Nitrils mit 3 bis 6 C-Atomen, eines Vinylhalogenids oder eines nichtaromatischen Kohlenwasserstoffs mit 4 bis 8 C-Atomen und mindestens 2 konjugierten Doppelbindungen, und
c) 0 bis 50 Gew.-% mindestens eines weiteren ethylenisch ungesättigten Monomeren besteht,
wobei der Gehalt des Monomeren a) so gewählt wird, daß das Copolymerisat zu 0,001 bis 20 Gew.-% aus Carbonylgruppen -CHO- oder Ketogruppen -CO- besteht und die Monomeren a), b) und c) sich zu 100 Gew.-% addieren.

6. Verwendung einer Dispersion oder Lösung nach einem der Ansprüche 3 bis 5 als Beschichtungsmittel.

7. Verwendung einer Dispersion oder Lösung nach einem der Ansprüche 3 bis 5 als Klebstoff.

8. Beschichtete Substrate, erhältlich unter Verwendung einer Dispersion oder Lösung nach einem der Ansprüche 4 bis 5.

9. Verklebter Verbund, erhältlich unter Verwendung einer Dispersion oder Lösung nach einem der Ansprüche 4 bis 5.

## Claims

1. A compound of the formula 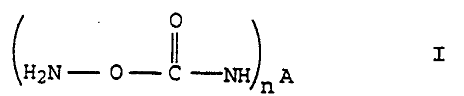 where A is an n-valent organic radical and n is an integer greater than 1, or a salt thereof.

2. A process for the preparation of a compound of the formula I, wherein polyisocyanates whose isocyanate groups are blocked by oximes or hydroxamic esters are reacted with water and an acid having an acid constant of > 10⁻².

3. A dispersion or solution of a free radical polymer, polycondensate or polyadduct, containing a compound of the formula I.

4. A dispersion or solution as claimed in claim 3, where the free radical polymer, polycondensate or polyadduct comprises from 0.001 to 20% by weight of aldehyde groups (CHO) or keto groups (CO).

5. A dispersion or solution as claimed in claim 3 or 4, containing a copolymer as a free radical polymer, wherein the copolymer consists of
a) at least one comonomer having at least one aldehyde or keto group,
b) from 20 to 99.99% by weight of at least one C₁-C₂₀-alkyl (meth)acrylate, one vinyl ester of carboxylic acids of 1 to 20 carbon atoms, one vinyl aromatic of up to 20 carbon atoms, one ethylenically unsaturated nitrile of 3 to 6 carbon atoms, one vinyl halide or one nonaromatic hydrocarbon having 4 to 8 carbon atoms and at least 2 conjugated double bonds, and
c) from 0 to 50% by weight of at least one further ethylenically unsaturated monomer,
the content of the monomer a) being chosen so that the copolymer contains from 0.001 to 20% by weight of aldehyde groups (CHO) or keto groups (CO), and the monomers a), b) and c) summing to 100% by weight.

6. Use of a dispersion or solution as claimed in any of claims 3 to 5 as a coating material.

7. Use of a dispersion or solution as claimed in any of claims 3 to 5 as an adhesive.

8. A coated substrate obtainable using a dispersion or solution as claimed in either of claims 4 and 5.

9. An adhesively bonded laminate obtainable using a dispersion or solution as claimed in either of claims 4 and 5.

## Revendications

1. Composé de formule 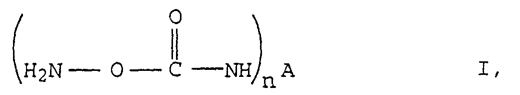 qui peut aussi se présenter sous forme de sel, A étant mis pour un reste organique à n valences et n étant un nombre entier supérieur à 1.

2. Procédé de préparation d'un composé de formule I, caractérisé en ce que des polyisocyanates, dont les groupements isocyanate sont bloqués par des oximes ou des esters d'acide hydroxamique, sont mis en réaction avec de l'eau et un acide ayant une constante d'acide supérieure à 10⁻².

3. Dispersion ou solution d'un produit de polymérisation radicalaire, d'un produit de polycondensation ou d'un produit de polyaddition, contenant un composé de formule I.

4. Dispersion ou solution d'un produit de polymérisation radicalaire, d'un produit de polycondensation ou d'un produit de polyaddition, qui se compose, pour 0,001 à 20% en poids, de groupements aldéhyde -CHO- ou de groupements céto -CO-, contenant un composé de formule I.

5. Dispersion ou solution selon la revendication 3 ou 4, contenant des copolymères en tant que produit de polymérisation radicalaire, le copolymère se composant
a) d'au moins un comonomère renfermant au moins un groupement aldéhyde ou céto,
b) de 20 à 99,99% en poids d'au moins un (méth)acrylate d'alkyle en C₁-C₂₀, d'un ester vinylique d'acides carboxyliques contenant 1 à 20 atomes de carbone, d'un composé vinylaromatique contenant jusqu'à 20 atomes de carbone, d'un nitrile à insaturation éthylénique contenant 3 à 6 atomes de carbone, d'un halogénure de vinyle ou d'un hydrocarbure non aromatique contenant 4 à 8 atomes de carbone et au moins 2 doubles liaisons conjuguées, et
c) de 0 à 50% en poids d'au moins un autre monomère à insaturation éthylénique,
la teneur en monomère a) étant choisie de sorte que le copolymère se compose, pour 0,001 à 20% en poids, de groupements carbonyle -CHO- ou de groupements céto -CO- et les teneurs en monomères a), b) et c) s'additionnant à 100% en poids.

6. Utilisation d'une dispersion ou solution selon l'une quelconque des revendications 3 à 5 comme produit d'enduction.

7. Utilisation d'une dispersion ou solution selon l'une quelconque des revendications 3 à 5 comme colle.

8. Substrats enduits, obtenus en utilisant une dispersion ou solution selon la revendication 4 ou 5,

9. Assemblages collés, obtenus en utilisant une dispersion ou solution selon la revendication 4 ou 5.
